# EUROPEAN PATENT APPLICATION

(11) **EP 3 960 714 A1**
(43) Date of publication of application: **02.03.2022**
(21) Application number: 20795573.3
(22) Date of filing: 23.04.2020
(51) Int. Cl.: C02F 1/68, C05F 11/00, C05D 9/00, A61K 33/00, A61K 47/12, A23L 33/10, A61P 3/02

(54) **SILICON ION COMPLEX ORGANIZED WITH CARBOXYLIC ACID, METHOD FOR MANUFACTURING COMPLEX, AND PRODUCT USING SAME**

(30) Priority: 24.04.2019 KR 20190047830; 28.02.2020 KR 20200025022
(71) Applicant: Korea New Technology Co.,Ltd., Seoul 07937 (KR)
(72) Inventor: YOON, Jong Oh, Incheon 21445 (KR)
(74) Representative: dompatent von Kreisler Selting Werner - Partnerschaft von Patent- und Rechtsanwälten mbB
(86) International application number: PCT/KR2020/005343
(87) International publication number: WO 2020/218834

(57) **Abstract**

The present invention relates to a technology related to the ionization of silicon, and more particularly, to a technology for ionization by organizing silicon using a watersoluble silicate with a tricarboxylic acid or a dicarboxylic acid.

This technology enables preparation and use of products containing an organized silicon ion complex in a variety of applications including foods such as water and beverages and medical products, as well as electrochemical applications. In particular, it is expected to treat and prevent various diseases caused by silicon deficiency by providing an organized form of silicon that does not exist as an ion in nature.

## Description

### TECHNICAL FIELD

The present invention relates to a silicon ion complex organized with a carboxylic acid, a preparation method for the silicon ion complex, and a product using the same.

### BACKGROUND ART

The generic term for elements contained in the human body or foods, excluding oxygen (O), carbon (C), hydrogen (H), and nitrogen (N), is mineral, also called ash in the past. About 96% of the elements in the human body are the four elements of O, C, H, and N, and only 4% are minerals. The minerals relatively high in quantity are calcium (Ca), phosphorus (P), potassium (K), sulfur (S), sodium (Na), chlorine (Cl), and magnesium (Mg), and the remaining trace elements are iron (Fe), copper (Cu), manganese (Mn), iodine (I), cobalt (Co), zinc (Zn), molybdenum (Mo), selenium (Se), chromium (Cr), fluorine (F), boron (B), arsenic (As), tin (Sn), silicon (Si), vanadium (V), nickel (Ni), titanium (Ti), and zirconium (Zr).

In other words, about 96% of the elements in the human body are organic substances consisting of oxygen (O), carbon (C), hydrogen (H), and nitrogen (N), and only about 4% are inorganic substances, generally called minerals.

The present invention relates to silicon (Si) among the minerals.

Silicon (Si), one of the minerals necessary for the human body, is known to play a certain role in memory loss, lack of patience, osteoporosis, aging, activation of metabolism, and prevention and treatment of aging in cells. But, not all functions of silicon have been fully identified.

According to a recent study at a UK university, silicon is not toxic. It is also known to be effective in suppressing cognitive decline in dementia patients.

If simply described, minerals existing in the form of ore are inorganic, and about 70 minerals are found in the earth.

These inorganic minerals are absorbed by plants in agriculture and combined with amino acids, and those in this state are organic. The minerals taken up by humans are mostly organic minerals absorbed from foods.

When ingested in an inorganic form, a mineral such as selenium (Se), for example, is not absorbed, but accumulated in the body to cause side effects. Hence, the selenium in an inorganic form can be normally absorbed only when combined with an amino acid and converted into an organic form. But, this does not apply to all minerals.

The same goes with the use of silica. Hence, silica in the ore form is processed into water-soluble silicon, which is dissolved in water and used for drinking purpose.

Such a water-soluble silicon is made to be soluble in water in the form of Na₂SiO₃, Na₂SiO₃·5H₂O, Na₂SiO₃·9H₂O, or Na₂SiO₃·10H₂O. In some cases, high-purity silicon dioxide and sodium carbonate as main raw materials are heated at high temperatures of 1,500 to 2,000 °C in the presence of a compound containing an element useful as a mineral, such as phosphorus (P), magnesium (Mg), and zinc (Zn), and agglomerated into a powder form.

That is, using silica as a digestible substance in plants, animals and humans has been achieved by the steps of (1) providing high-purity silicon oxide as a mineral; (2) converting the silicon oxide into a water-soluble silicon compound by synthetic reaction with sodium carbonate or sodium hydroxide at high temperature; and (3) dissolving the water-soluble silicon compound. Yet, the water-soluble silicon compound upon reacting with water does not remain in an ionic state, but takes a colloid or colloidal form.

The water-soluble silicon is very highly alkaline and stable in alkali; but, in reality, it begins to form a fine-sized gel and eventually precipitates through agglomeration of particles.

The water-soluble silicon thus obtained or the water-soluble silicon compound containing the water-soluble silicon as a main component is stable only in alkali, not in acids or alcohols, and not able to remain as ions.

This makes the water-soluble silicon or the water-soluble silicon compound difficult to digest and absorb in the digestive system of animal, plant, or human bodies. It is therefore necessary to maintain it in an ionic state without sedimentation throughout the digestive system beginning from the stomach.

In addition, in order to acquire the availability of silicon in various industrial applications, there has emerged the necessity of systematical study on the development of the technology to increase the stability of ions in aqueous solutions, acidic or alkaline.

Minerals are mainly mineral substances contained in ores (especially biotite), including germanium needed to make a semiconductor, and other well-known elements such as iron, manganese, etc. The minerals contained in stone or rock masses are gradually dissolved in the underground water flowing through the ground and absorbed into the ground.

These minerals are essential to all living things. However, it is no exaggeration to say that almost all the lands on the earth are currently depleted of minerals, because more minerals are absorbed and lost by plants in large-scale agriculture than those gradually seeping into the ground.

When the soil lacks these minerals, plants cannot absorb sufficient nutrients even with different types of fertilizers given abundantly, resulting in weak roots, slow growth and low yield of harvest.

Another serious issue is the collapse of the soil mineral cycle due to chemical farming. The soil mineral cycle is maintained by organic fertilizers such as compost and livestock manure. The agricultural produces harvested from the soil of which the mineral cycle is blocked are bound to be deficient in minerals.

The mineral cycle of the modern human body, which has no choice but to consume such agricultural produces, is also bound to collapse. The biggest cause of adult diseases and half-health condition of the modern people can be considered the deficiency of the minerals.

According to the warning of World-renowned health scholars such as Bragg, Walker, and Dr. Vannick, minerals must be absorbed through foods (animals or plants); otherwise, it may damage the central organs of the human body and cause various diseases if inorganic minerals in water or a solution of naturally occurring minerals diluted with water are taken.

Many of the minerals contained in the current medicines are inorganic minerals. They are, of course, intended for intake of minerals as needed and not harmful to the human body. The minerals absorbed into the human body, whether organic or inorganic, are temporarily ionized and then recombined into necessary organic substances, which are acting as enzymes in some parts of the body or being deposited in tissues. That is, the minerals need to be ionized at least for digestion (absorption) into the human body. Due to the nature of minerals, there are cases in which organic substances are more absorbable in the absorption process.

Accordingly, it is necessary to organize and ionize minerals, even if they are inorganic.

### SUMMARY OF THE DISCLOSURE

It is therefore an object of the present invention to provide a silicon ion complex organized with a carboxylic acid using a water-soluble silicon compound.

It is another object of the present invention to provide a technology for preparing a silicon ion complex organized with a carboxylic acid that includes organizing a water-soluble silicon compound to prepare a silicon ion complex for use in animals including human and the growth of plants.

It is further another object of the present invention to provide a method for adding titanium, zirconium, or various minerals containing titanium or zirconium in preparation of the silicon ion complex organized with a carboxylic acid that is stable to changes in hydrogen ion concentration (pH).

It is still further another object of the present invention to provide various products using the organized silicon ion complex prepared by using the preparation technology for the silicon ion complex organized with a carboxylic acid.

The inventors of the present invention have been continuously developing a technology for stabilization of titanium- or zirconium-related ions in an aqueous solution and also studying the ionization of various substances as well, filing invention patents using various titanium- or zirconium-related ions since 2011.

Meanwhile, looking at the prior art documents of the present invention, there are listed various technologies for ingestion of minerals, such as silicon or zirconium, application of the minerals to plants or animals, and drinking of the minerals.

Particularly, a silicate beverage containing zirconium and a preparation method for the same is described in the claims and contents of Korean Patent Application No. 10-2017-0034531 (Patent Document 0015).

Besides, Korean Patent Application No. 10-2002-0071408 discloses a liquid composition for acceleration of plant growth that contains fine particles of titanium dioxide.

The common point of silicon, zirconium, and titanium intended to be applied to animals, plants, and human body is that they are non-toxic as minerals.

Zirconium, of which the functions in living organisms are still not clear, is contained in an amount of about 250 mg on average in the human body and also in small amounts in various foods. In daily life, it is also used in some products or for purification of tap water (Source: Wiki Encyclopedia).

Titanium or silicon has been used as a plant growth accelerator or a liquid fertilizer for plants and recognized as a mineral in the human body. The role of titanium in the human body has not yet been identified, but titanium is found in trace amounts in the human body.

Each of these elements has different properties.

When a water-soluble zirconium compound is added into water, for example, zirconium tends to be stable at low hydrogen ion concentration below pH 4.5 and precipitates in alkali. The zirconium remains in an ionic state without any special additive at low pH, but abruptly becomes unstable with an increase in the pH and completely precipitates in alkali.

When a water-soluble titanium compound such as titanium tetrachloride or titanium sulfate is added into water, titanium reacts with water to produce a precipitate in a very short time (e.g., less than 1 second), so it is unable to exist in the form of ions in water. The titanium reacts with water very quickly irrespective of whether the titanium is in acid or alkali. Of course, titanium, if in the form of ions, is more stable in acid than in alkali.

In addition, when a water-soluble silicates, e.g., sodium silicate, potassium silicate, or calcium silicate, are dissolved in water, the solution becomes alkaline normally with a pH of 10.0 to 13.0 depending on the amount of the added water-soluble silicates. These substances that are dissolved in water to form an aqueous solution happen to react with water with an elapse of time to increase in weight and volume, ending up precipitating at a low pH level or, if left for a long time, producing a precipitate due to their water-absorbent property even after dissolution. Particularly, when in acid with a low pH level or in alcohol, the substances become insoluble.

It is like the water-absorbent property of silica gel.

More specifically, Korean Patent Application No. 10-2016-7021929 discloses a stabilized polysilicate. The stabilized silicate composition according to the cited patent has properties very suitable for use as an antiperspirant, shows such a high safety as the polysilicic acids acceptable to the skin do and, unlike aluminum salts, has no safety issue concerning the systemic toxicity.

Moreover, as the pH (2.5 or 3.0 to 5.0) of the initial composition is changed to the pH suitable for gel formation, i.e., pH 6, which is a physiologically acceptable condition, the gel-forming reaction occurs in a short time, typically between 5 and 30 minutes. In the cited patent, the gel produced by the pH change of the composition is odorless and colorless and leaves no noticeable stains on the skin or clothing.

The invention provides a method for producing a stabilized polysilicic acid that includes the steps of:
(a) preparing an alkaline silicate solution having a pH of 9.5 or above;
(b) selectively adding a growth retardant to the alkaline silicate solution;
(c) lowering the pH to 4.0 or below by addition of an acid to form a composition containing a polysilicic acid;
(d) selectively adding a multivalent cation;
(e) increasing the pH of the composition to a physiologically acceptable pH value by addition of a base to form a stabilized composition containing a polysilicic acid;
(f) selectively adding a growth retardant capable of increasing the safety of the composition;
(g) selectively adding a non-aqueous solvent capable of increasing the safety of the composition; and
(h) selectively introducing the composition into an antiperspirant composition or a cosmetic formulation.

The invention also provides a stabilized silicate composition obtainable by the disclosed technology that includes the step of producing a stabilized silicate composition in addition to the step (c) of lowering the pH to 4.0 or below by addition of an acid to form a composition containing a polysilicic acid.

In summary, Korean Patent Application No. 10-2016-7021929 has an object to cause a gel-forming reaction at a pH value of 2.5 to 3.0, pH 5.0, and approximately pH 6.0, which is a physiologically acceptable condition, and another object to adjust the gel state of the silicate to have an average size of 20 nm or less.

Referring to the specific details for carrying out the invention, the invention is directed to a silicate in the nanoparticle or colloid form of polysilicic acid with a cluster radius of less than 10 Å, and preferably to a colloidal dispersion solution containing nanoparticles of a polymeric silicic acid with an average diameter of less than 100 nm, specifically between 1 to 100 nm, and preferably 20 nm.

The reason the silicic acid has such a variety of particle sizes in an aqueous solution is the water-soluble silicate having a water-absorbent property while dissolved in water. Such a water-absorbent property is the reason silica gel acts as a dehumidifying agent.

That is, the water-soluble silicate, if first dissolved in water without any action, reacts with water to form a gel during the dissolution process and cannot get back to ions in the aqueous solution.

On the other hand, Korean Patent Application No. 10-2016-7024827 (Patent Document 35) is similar to the present invention in terms of the use of the materials and method related to a stabilized polymeric silicate composition.

Particularly, Paragraph [0056] specifies the use of the same carboxylic acid as used in the present invention. In addition, paragraph [0058] describes that ethanol is expected to act as a stabilizing agent to control the water-absorbent property of the polymeric silicate composition.

This invention has an object to prepare a stabilized polymeric silicate composition comprising a polymeric silicic acid and silicate nanoparticles with an average diameter of 20 nm or less. The term "stabilized" refers to maintaining the average diameter and size of the particles without any increase. In this regard, it is similar to Patent Document 34 in a certain aspect.

The invention describes the preparation of a stabilized silicate composition that includes the steps of: (a) providing an aqueous solution of a soluble silicate at pH of 9.5 or above; (b) lowering the pH level of the silicate solution to cause a polymerization of silicate and form a polymeric silicic acid and silicate nanoparticles; and (c) adding a stabilizing agent including polyalkylene glycol and/or sugar to the silicate solution at the same time as or sequentially after the step (a) and/or the step (b).

That is, the invention causes polymerization to form a silicic acid and silicate nanoparticles and uses a stabilizing agent for the sake of stabilizing the size of the particles, where the stabilizing agent is a carboxylic acid as specified in paragraph [0056].

It seems that the inventors of this invention has neither a purpose of making silicon into ions from the beginning nor a knowledge about ionization of silicon at the time of invention.

The inventors of the Patent Document 35 claim in paragraph [0068] that the stabilized polymeric silicate composition is intended for use as a pharmaceutical or nutritional composition.

As well known to those skilled in the art, the safest way to take up minerals, such as silicon, as a nutrition by humans or animals is to ingest them in the form of ions.

Particularly, any one can find out the data easily accessible through various media suggesting that in terms of a method of ingesting minerals, either inorganic or organic (chelated), ingestion of inorganic minerals can be harmful to the human body, even if they are in the form of ions. Data about inorganic minerals can be easily obtained just by Internet search.

What does 'chelate' or 'chelated' mean?

The word, chelate clearly describes the appearance of chemical bonds formed in a way that can be compared to the grasping of an object by a crab's claw.

Minerals are a type of mineral matters and also described as mineral or inorganic because they are extracted from ore. The minerals used for animal feeds are extracted from ore and also called inorganic minerals. But, minerals exist in a different form in the animal or plant body. Inorganic minerals are consumed by animals or plants and combined with an organic substance via a chelating bond in the animal or plant body, to form so-called chelated minerals.

In this situation, silicon has a material property that it cannot be made into an inorganic mineral unless undrinkable hydrofluoric acid (HF) is used. Hence, the method of using silicon as an ion has been completely impracticable. In other words, even inorganic minerals introduced into the human body can dissolve in gastric acid and become digestible/absorbable in the form of ions. Of course, organic minerals can be for sure ingested in a safer way.

However, silicon becomes polymeric silicate particles, which are polymer materials that absorb water when in contact with water and increase in volume and weight. It is therefore a challenge in the related industry to maintain silicon in an ionic state by inhibiting the water-absorbent reaction.

SiO₂, present in a trace amount in natural water, may not have a great adverse effect on the health in the human body. But, the fact that silicate polymer particles that are a polymer substance act as a nutrient in the human body is just the inventors' claim and considered incomprehensible to the developers who have long been studying minerals so far.

The minerals chelated with amino acids are 3 to 4 times higher in absorption and bioavailability than any form of minerals existing in nature. The reason the chelated minerals have high absorption and bioavailability is a different digestion and absorption mechanism. Inorganic minerals consumed by livestock are supposed to undergo a complicated ion absorption process in which metal ions produced by ionization form a chelating bond to the integral proteins present in the intestinal membrane, become separated from the integral proteins and create a chelating bond to carrier proteins. In contrast, chelated minerals are absorbed not through such a complicated ion absorption process but through an active absorption process in which they are absorbed along with amino acids directly into the intestinal membrane cells through the absorption passage of dipeptide.

Further, the inorganic minerals in the stomach are separated into ions, which are divalent cations. The divalent cations combine with anionic phosphoric acid, oxalic acid, phytic acid, and fibrin in the intestine, to form indigestible substances, resulting in a significant reduction of the absorption and bioavailability.

In the cited invention of Patent Document 35, which is a patent related to silicon, the silicate polymer that is a polymer substance is not separated into ions in the digestive system, human or other animal's stomach. Therefore, it seems that the inventors have developed or suggested the use of the silicon polymer as a nutrient without the knowledge that a polymeric silicate composition converted from tetravalent silicon cannot be broken into ions in the gastric acid solution.

Inorganic minerals break up into ions in the stomach, so they can be used for medical purposes or added to animal feeds, in case of necessary. Yet, using polymer substances for such use purposes, even in trace amounts, may have unknown effects on the human or other animals and hence is considered inappropriate as a way to take up minerals.

Thus, it may be an extremely unusual claim, except for natural polymer substances such as starch and cellulose, that living organisms can drink and ingest polymer substances even in small amounts.

The inventors of the present invention have succeeded in maintaining organized titanium as ions by dissolving a water-soluble titanium compound such as titanium tetrachloride or titanium sulfate in a solution of tricarboxylic acid or dicarboxylic acid and maintaining organized zirconium as ions by dissolving a water-soluble zirconium compound such as zirconium oxychloride or zirconium sulfate in the same manner as described above, in the conventional method of organizing titanium and zirconium into ions.

It was proved through experimental examples and examples and confirmed as a registered patent that metal ions can be present in the form of organic ions in an aqueous solution through plating of various alloys and single metals. Particularly, it was also proved that metals can be used for electrochemical applications through a chelating method even when they are present as ions in an aqueous solution.

In other words, through the oxidation and reduction of metals by electricity in electroplating, it was reliably proved by way of the behavior of metal ions that titanium and zirconium can exist as organized ions by combining with a carboxylic acid.

In this process, it can be seen from an experiment that the stability of the organized ions can be more improved by adding a plurality of carboxylic acids rather than a single carboxylic acid.

### [Experimental Example 1] Experiment 1 for Stabilization of Tungsten Ion

Experimental Example 1 and 2 were performed to understand the behavior of ions in an aqueous solution of silicate through an experiment for the stabilization of tungsten ions that are stable in alkali similarly to water-soluble silicates.

18 g of sodium tungstate and 18 g of citric acid as a tricarboxylic acid were dissolved in 100 ml of water at 60 °C.

The resultant solution was clear at the time of dissolution. In 48 hours of a storage test, the solution began to form a sponge-like precipitate with tungsten ions agglomerating and precipitating. Therefore, the re-dissolution of the precipitate was much impossible to carry out: the precipitate may be re-dissolved by separation from the aqueous solution and reaction with ammonia; yet, if it is a silicate, its re-dissolution is impracticable.

Assuming the dissolution state of the silicate, the stability of the tungsten ions, stable in alkali, at pH 3.0 was investigated. But, the tungsten ions were not completely stabilized with a tricarboxylic acid. The amount of the tricarboxylic acid might be insufficient.

Like this, different variables may act on the ionization of substances.

According to the inventors' observation, the water-soluble silicate appear to be stable in alkali, but in reality, it is unstable in alkali and just slow in reacting with water, showing similarity in behavior of ions to the metal, tungsten that is stable in alkali. In reality, the water-soluble silicate is present in the form of a colloid or colloidal form rather than an ion, but looks like an ion as the colloid is too clear to recognize with the naked eye. It is possible to determine whether it is an ion or not, according to a high-purity ethanol reaction test. Upon added to a high-purity ethanol, the aqueous solution of silicate in the colloid or colloidal form turns from clear to cloudy, clearly showing its particle form.

In regards to the actual form of silicate, upon in contact with water, the silicate immediately starts to react with water as titanium does and absorbs water molecules due to its endothermic and water-absorbent properties to expand its volume and weight. When expanded, it is unable to return to the original ionic state in the defined solution merely by a method of changing the pH level or the like.

Among metals, zirconium and titanium have the similar property that a precipitated substance in the defined solution is unable to return to the original ionic state. When they are out of the ionic state in water and form a precipitate, they cannot get back to the ionic state in the defined solution by pH adjustment unless a highly toxic substance such as fluorine is used. Tungsten in the previous experimental example was able to return from a precipitate to an ion by dissolution in ammonia, but titanium and zirconium were not.

The precipitate needs to be removed by filtration.

The water-soluble silicates are not able to return from the colloid or colloidal form to ions in an aqueous solution. This is similar in the reason to the case that a precipitated titanium becomes hydrated titanium oxides and is thus impossible to dissolve.

In other words, the water-soluble silicate appeared to have a stable appearance in alkali, similarly to a metal such as tungsten stable in alkali. But in reality, it had a slight decrease in the rate of activating the water-absorbent property when in contact with water and, upon break-up of the ionic state, it existed in a colloid or colloidal form, causing a polymerization at a low rate, and was unable to return to the ionic state.

The method by which the inventors of the present invention have been able to maintain titanium or zirconium in the ionic state in an aqueous solution involves preparing an aqueous solution of dicarboxylic acid or tricarboxylic acid, dissolving a water-soluble titanium compound or a water-soluble zirconium compound in the aqueous solution, and causing the temporarily produced metal ions to combine with the carboxylic acid, thereby organizing the metal ions. For this, the carboxylic acid must be dissolved first of all.

Similarly to titanium or zirconium, a water-soluble silicate can also be maintained in the ionic state through organization by a method that involves preparing an aqueous solution of dicarboxylic acid or tricarboxylic acid, dissolving a water-soluble titanium compound or a water-soluble zirconium compound in the aqueous solution, and, prior to precipitation, causing the ionic form of the water-soluble silicate dissolved in the aqueous solution to combine with the carboxylic acid in the same manner of the metal ions forming a complex with a carboxylic acid, thereby contriving the present invention.

The process of dissolving the carboxylic acid prior to the water-soluble silicate is the core of the technology to maintain the water-soluble silicate in the ionic state by creating a bond to the carboxylic acid before the water-soluble silicate takes a colloid or colloidal form larger than ions.

### [Experimental Example 2] Experiment 2 for Stabilization of Tungsten Ion

18 g of sodium tungstate, 18 g of tartaric acid as a tricarboxylic acid, and 9 g of citric acid were dissolved in pure water to make 100 ml of a solution.

The resultant solution was clear at the time of dissolution. In 48 hours of a storage test, it was stable and remained clear.

The pH of the solution was adjusted several times with ammonia and sulfuric acid in a range from pH ≤ 1.0 to pH ≤ 11.0, but the solution remained stable.

It can be seen from this experiment that a combination of different carboxylic acids helps organize the ionic substances and stabilize the pH level. Particularly, the more the carboxyl groups, the more helpful in stabilizing the ionic substances in an aqueous solution.

The ionic substances can be more stabilized in an aqueous solution by using a combination of tricarboxylic acids or a combination of tricarboxylic acids and dicarboxylic acids. That is, tricarboxylic acids are preferred to dicarboxylic acids for stabilization of ions.

For example, ions can be a little more stabilized in an aqueous solution by using a combination of malic acid and citric acid; glycine and malic acid; glycine, citric acid and malic acid; tartaric acid and glycine; tartaric acid and malic acid; tartaric acid, citric acid and glycine; tartaric acid, malic acid and glycine; or tartaric acid and citric acid.

In other words, a water-soluble silicon compound, a water-soluble titanium compound, or water-soluble zirconium compound can be organized and stabilized in an ionic state by binding with organic acids using a combination of dicarboxylic acid and tricarboxylic acid, such as glycine, citric acid, malic acid, tartaric acid, or succinic acid. If a single organic acid is used, a tricarboxylic acid is preferred to a dicarboxylic acid.

In addition, the pH may be adjusted unrestrictedly to a value from 1.0 or below to 13.0.

Such a change in pH can be achieved to have all materials ingestible and not harmful to animals including humans and plants.

Through this process, silicon ions can be organized and remain as ions in an aqueous solution.

The inventors of the present invention have found it out that the order of silicon organization is very important as much as that of titanium/zirconium organization to maintain the ionic state, thereby contriving the present invention.

The water-soluble silicon compounds dissolve in water, and upon dissolution, immediately react with water to have an increase in weight and mass to a certain extent, thus increasing the amount of precipitates depending on the pH level. The precipitate is not observed for a longer time in strong alkali, and immediately it appears as completely insoluble to water at a low pH value. The gel thus formed is unable to be re-ionized in the aqueous solution.

For example, the water-solution silicates in the nonionic state do not break up into ions, but they mostly precipitate during digestion at pH 1.5 or below that corresponds to human gastric acid. Due to this, the water-soluble silicates completely dissolved in alkali become gelled into a silica gel form at a lower pH. Therefore, no matter how completely the water-soluble silicates are dissolved in alkali, it is not certain that the water-soluble silicates can be maintained in an ionic state throughout the digestive system beginning from the stomach in the animal or human body.

It was, however, discovered that when the water-soluble silicates form complexes while dissolved in water in which dicarboxylic acids or tricarboxylic acids are dissolved first of all, they can be stabilized in the ionic state without water absorption, thereby contriving the present invention.

That is, water-soluble silicates are disabled to have the water-absorbent property any more if forced to react with a carboxylic acid immediately (i.e., in a short period of time of being present as ions) after dissolution in water. This means that the water-soluble silicates form complexes with the carboxylic acid and are present in the form of the smallest ions rather than reacting with water and being dispersed or present in the colloidal form.

The inventors of the present invention has thus so far confirmed that substances can be organized and made into ions and that titanium and zirconium can also be organized and maintained in an ionic state in the same way. Studies have been made on the process of metal oxidation and reduction by electroplating in order to demonstrate that the metal is in the ionic state, which is made out by the invention patent.

If not demonstrated by the process on the ground of silicon being not a metal, it can be indirectly explained that silicon can form a complex with a carboxylic acid and remain in an ionic state through the conventional process used for titanium and zirconium.

### (Definition of Terms)

The term "silicon ion complex" as used in the present invention will be described as follows.

In order to make silicon exist as ions, minimal essential components are required.

Such essential components are needed due to the various properties of silicon as follows.

Firstly, silicon is soluble in hydrofluoric acid, which is a highly toxic substance and hence difficult and dangerous to use for industrial purposes. The same goes for titanium and zirconium. Even at the time of the present invention, the inventors of the present invention could find no scientific evidence that silicon dissolves in boiling sulfuric acid, although a very small amount of titanium can dissolve in boiling sulfuric acid as well as hydrofluoric acid.

Secondly, silicon becomes soluble in water when converted into sodium silicate as a sodium-silicon composite substance through a synthesis reaction with sodium carbonate or sodium hydroxide; yet, similar tetravalent metals, titanium and zirconium do not dissolve in water even when combined with sodium; that is, every tetravalent metal has different properties.

Thirdly, even though processed into sodium silicate, silicon remains in an ionic state in water only for a very short period of time upon dissolution in water. The duration of the ionic state is very difficult to measure due to the silicon itself being almost clear. Upon in contact with water, titanium and zirconium also remain as ions for a very short period of time less than about one second and then start to precipitate again. Silicon in contact with water does not precipitate, but immediately turns to a colloid or colloidal form for a very short period of time, gradually absorbing water, maintaining the colloidal state, agglomerating, and sedimenting as an insoluble precipitate over time; it is considered a polymeric silicate as a polymer).

Due to the properties of silicon, in the course of obtaining silicon ions that are safe as ions touchable and consumable by humans, "sodium" in the form of sodium carbonate or sodium hydroxide is used to synthesize sodium silicate for dissolution of silicon in water. First of all, it is necessary to dissolve at least one selected from tricarboxylic acids and dicarboxylic acids in water so that the sodium silicate can exist as ions while dissolved in water. Hence, "sodium", "silicon", "carboxylic acid', and 'water" must be included as essential components in order for the sodium silicate to exist as ions. For this reason, the term unavoidably chosen is "silicon ion complex". It is due to the characteristic of the substance that it does not exist as ions in water without an organic substance as sodium chloride does.

The same goes for potassium and calcium, so the same result is obtained when potassium silicate or calcium silicate are used.

First, an aqueous solution of carboxylic acid is prepared using a combination of carboxylic acids selected from dicarboxylic acids and tricarboxylic acids. Then, a water-soluble silicate is dissolved in the aqueous solution of carboxylic acid. Therefore, when in contact with water or under low pH conditions, silicon is able to exist as an organic ion, which is composed of silicone bonded to an organic acid.

This can be confirmed through an experiment to determine whether silicon maintains a stable state in the pH range between pH 1.5 that is similar to the pH of the gastric solution and pH 13.0. To be more stable against changes in pH, silicon can be made to maintain in an ionic state without gelation or precipitation even at pH ≤ 1.0.

This is very important.

In general, silicon is easily considered as a substance not edible for people. But in reality, the actual surface of the earth crust is rich in silicon, and humans have been ingesting silicon through plants.

As far as it is known, silicon deficiency in the human body may cause poor formation of ankle and knee joints, decreased glycosaminoglycan content in articular cartilage, osteoporosis, lack of elasticity in connective tissues, skin aging, hair and nail damage, and so forth.

Also, the physiological functions of silicon in the human body are known as follows:
(1) Binds to the carbon chain of collagen, elastin, mucopolysaccharides, hyaluronic acid, etc. and involves crosslinking;
(2) Involves construction or elasticity of connective tissues;
(3) Accelerates bond calcification (high silicon content in the site where bone is highly calcified);
(4) Plays an essential role in production of collagen and promotes calcium absorption in the initial stage of bone formation (preventing osteoporosis);
(5) Makes nails, hair and skin elastic and glossy;
(6) Maintains the elasticity of arterioles (preventing cardiovascular diseases);
(7) Prevents accumulation of aluminum in the body (preventing Alzheimer's diseases); and
(8) Needed to maintain the right functions of prolyl hydroxylase, an enzyme that functions in the formation of bone and other connective tissues cartilage.

The clinical efficacies of silicon are known to help prevent/treat/maintain osteoporosis, osteroarthritis, rheumatoid arthritis, skin health and skin aging, arteriosclerosis, nails/hair health, Alzheimer's disease, and so forth.

Although not clear, refined foods are often consumed to cause a low intake of silicon in civilized countries with a lot of arteriosclerotic cases. Those who consume fiber foods rich in silicon have a low serum cholesterol level and low arteriosclerosis. Yet, the silicon content in the aorta decreases with age in general.

Nevertheless, silicon does not exist as an element in nature, but as oxide forms (e.g., silica). Hence, the humans are able to ingest silicon as an element combined with amino acids by way of the fiber of plants, especially fiber-rich plants.

One of the foods with the highest amount of silicon is alfalfa, which is very rich in calcium, magnesium, potassium, and silicon and thus often used against digestive ulcers, gastritis, liver disease, eczema, hemorrhoids, asthma, high blood pressure, anemia, constipation, bleeding gums, inflammation, burns, cancer, swelling, etc.

It is reported that silicon supplementation increases the thickness of the dermis right beneath the skin surface and the support structure for connective tissue.

It is problematic that the silicon available for consumption as fiber from the nature is running out. Yet, the present invention takes over the work of plants to organize silicon and to provide the organized silicon in the form of an ionized complex.

Meanwhile, carboxylic acid compounds are collectively referred to as the general formula RCOOH.

Carboxylic acid is a kind of organic compound, and its liquid is mostly acidic. Almost all compounds of the carboxyl group have a suffix of "-acid".

Unlike other hydrocarbons, large-scale compounds are chemically meaningful, and representative ones are amino acids and fatty acids.

Desired results can be obtained through various types of reactions with different compounds having carboxylic groups according to needs, such as for promoting plant growth, including fertilizers used in agriculture, for animals, for human medicine, and for cosmetics.

The name carboxylic acid is given because the hydrogens of the carboxyl group in these compounds having carboxyl groups dissociate to show acidity.

In classification of carboxylic acids in this way, those having one carboxyl group are referred to as monocarboxylic acids, those having two carboxyl groups are referred to as dicarboxylic acids, and those having three carboxyl groups are referred to as tricarboxylic acids.

Chain-shaped monocarboxylic acids are widely distributed as a component of fats or oils, so they are especially called fatty acids. The fatty acids play a role as additives, but water-soluble silicates, titanium ions, and zirconium ions are difficult to stabilize in an aqueous solution.

Accordingly, in the present invention, the compound having carboxyl groups capable of stabilizing a water-soluble silicate compound may be properly at least one carboxylic acid selected from tricarboxylic acids and dicarboxylic acids, more preferably tricarboxylic acids.

Accordingly, the present invention may further include stabilizing a water-soluble silicate compound using at least one tricarboxylic acid, preparing an aqueous solution of carboxylic acid by dissolving at least one dicarboxylic acid or tricarboxylic acid under necessity, and then dissolving a water-soluble silicate compound, a water-soluble titanium compound, or a water-soluble zirconium compound in the aqueous solution of carboxylic acid.

In this regard, the dicarboxylic acid or tricarboxylic acid is an amino acid.

In preparing an acidic aqueous solution using a water-soluble silicate, an aqueous solution of carboxylic acid is first prepared by dissolving at least one dicarboxylic acid or tricarboxylic acid, and then a water-soluble silicate compound is dissolved in the aqueous solution of carboxylic acid to prepare an organized silicon ion complex; the water-soluble silicate is made to form a complex with a carboxylic acid at low temperature while the rate of reaction between the water-soluble silicate and water is lowered as much as possible.

In order to add titanium and zirconium, under necessity, an aqueous titanium ion solution and an aqueous zirconium ion solution are added to the organized water-soluble silicate ion, where the aqueous titanium ion solution is prepared as a solution of organized water-soluble titanium ions by dissolving a water-soluble titanium compound in an aqueous solution of carboxylic acid prepared by dissolution of at least one dicarboxylic acid or tricarboxylic acid; and the aqueous zirconium ion solution is prepared as a solution of organized water-soluble zirconium ions by dissolving a water-soluble zirconium compound in an aqueous solution of carboxylic acid prepared by dissolution of at least one dicarboxylic acid or tricarboxylic acid.

Accordingly, the present invention provides a method for producing a silicon ion complex organized with a carboxylic acid using a water-soluble silicate compound that includes the steps of:
(1) dissolving at least one carboxylic acid selected from a dicarboxylic acid or a tricarboxylic acid in distilled water or drinkable clean water first of all to prepare an aqueous solution of carboxylic acid;
(2) adjusting the pH of the aqueous solution of carboxylic acid to acid and alkali under necessity;
(3) dissolving a water-soluble silicate compound in the pH-adjusted aqueous solution of carboxylic acid; and
(4) selectively adding a polyvalent ion to prepare an organized silicon ion.

The method may further include the step of adding an organized titanium ion solution and an organized zirconium ion solution in the step (4).

The method may further include the step of adding an ion used as a mineral under necessity in steps (1), (2) and (3) .

Here, the added ion is magnesium (Mg), potassium (K), iron (Fe), manganese (Mn), sodium (Na), zinc (Zn), sulfur (S), calcium (Ca), and phosphorus (P).

According to the present invention, a water-soluble silicate can be ionized into a silicon ion complex, which is drinkable and used to various industrial applications.

In particular, the silicon ion complex of the present invention can be used as a source of minerals for plants, animals, and humans lacking minerals and applicable in medicinal and pharmaceutical fields. Further, the present invention can solve the problem with the prior art that the colloid or colloidal form is difficult to absorb through the digestive tract and thus produce an organized ion form digestible and absorbable.

### BRIEF DESCRIPTION OF THE DRAWINGS

FIG. 1 shows a silicate precipitated in a gastric acid environment (pH 1.5 or below).
FIG. 2 is a diagram showing the crystal structure obtained through evaporation of a saturated solution of an organized silicon ion complex prepared with tartaric acid.
FIG. 3 is a diagram showing the crystal structure in a saturated solution of an organized silicon ion complex prepared with tartaric acid.
FIG. 4 shows the surface of an accessory product plated with a gold plating solution containing the organized silicon ion complex prepared with tartaric acid in Example 3.
FIG. 5 shows a substitution reaction test performed using the silicon ion complex organized with tartaric acid as prepared in Example 3 on the surface of a stainless steel product (left: the stainless steel surface before substitution reaction, right: the stainless steel surface after substitution reaction).
FIG. 6 shows an experimental image showing water repellence on a stainless steel surface after substitution reaction with silicon ions.

### DETAILED DESCRIPTION OF THE DISCLOSURE

Hereinafter, the present invention will be described as follows.

In order to explain a silicon ion complex organized with a carboxylic acid according to the present invention, there was performed an experiment for ionization through organization of a water-soluble silicate using (1) an organic acid having a tricarboxyl group or a dicarboxyl group, such as citric acid, malic acid, or tartaric acid and (2) glycine as one of amino acids.

The water-soluble silicate used to explain the present invention is Na₂SiO₃·9H₂O, but is not limited thereto. It may also be Na₂SiO₃, Na₂SiO₃·5H₂O, Na₂SiO₃·10H₂O, and mineral-containing water-soluble silicate complexes produced in the preparation of a water-soluble silicate. Hydrates containing water molecules in the molecule have some potential to form insoluble precipitates, but they can exist as ions without any specific problems when dissolved at a low temperature. In general, water-soluble silicate complexes containing various mineral components are produced in the preparation process for water-soluble silicates, and the mineral components chiefly available in the process may include magnesium (Mg), potassium (K), iron (Fe), manganese (Mn), sodium (Na), zinc (Zn), sulfur (S), calcium (Ca), and phosphorus (P).

These mineral components are used by way of adding substances such as sodium carbonate, potassium carbonate, sodium triphosphate, sodium pyrophosphate, sea salt, magnesium carbonate, calcium carbonate, magnesium oxide, calcium oxide, iron oxide, or manganese oxide.

Hereinafter, the present invention will be described in detail with reference to the specific examples as follows.

### [Comparative Example 1] Experiment 1 for Metasilicate Dissolution

A dissolution experiment was performed using sodium metasilicate nonahydrate (Na₂SiO₃·9H₂O).

The procedures were performed as follows.
(1) 1 g of metasilicate nonahydrate (Na₂SiO₃·9H₂O) was dissolved in 500 ml of pure water to prepare a solution, which was clear and had a pH of 12.23 (with a measurement deviation of a digital pH meter).
(2) The pH of the solution was lowered to pH 1.5 using 37% hydrochloric acid and measured again.
(3) The dissolved silicate gelled while agglomerating.

### [Comparative Example 2] Experiment 2 for Metasilicate Dissolution (Gastric Acid Conditions)

(1) 150 ml of water was added in a 250ml beaker, adjusted in pH to a value of 1.5 with 37% hydrochloric acid to meet the standards for gastric acid of the human body, and combined with another water to a volume of 250 ml.
(2) 1 g of metasilicate nonahydrate (Na₂SiO₃·9H₂O) was added to the solution of step (1).
(3) The silicate got partly loose, but precipitated rather than dissolved.

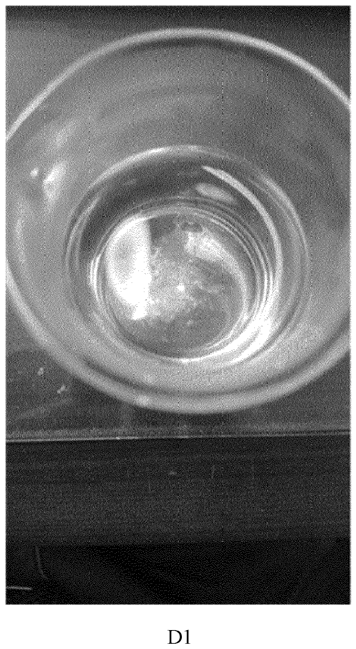

Through the comparative examples, it was confirmed that the metasilicate was unable to exist in an ionic state alone in the gastric acid (FIG. 1).

Even though the metasilicate was reduced to a nanoscale size, it was in the form of silica gel, which was difficult to digest and absorb.

The precipitated substance was unable to re-ionize in the same solution.

### [Example 1] Experiment for Preparation of Organized Metasilicate Ion Complex Using Citric Acid

An organic ionization testing was performed using metasilicate nonahydrate (Na₂SiO₃·9H₂O).

The procedures were performed as follows.
(1) 20 g of citric anhydride was added into a 500ml beaker.
(2) Sodium carbonate was used to adjust the pH to a value of 8.0 (alkaline); this pH adjustment was conducted to prevent insoluble impurities from produced by pH shock, due to the metasilicate dissolving easily under neutral-to-alkali conditions without pH shock.
(3) 20 g of silicate nonahydrate (Na₂SiO₃·9H₂O) was slowly dissolved under mechanical agitation; water absorption and endothermic reaction occurred during the dissolution process.
(4) The organized silicon ion complex thus obtained was stabilized in the form of a clear solution. 20 g of the complex was sufficiently dissolved in 500 ml of the solution and maintained in an ionic state; when reduced to the amount of pure silicon ions, the silicon ion content amounted to about 3.954 g/L.
(5) 100 ml of the organized silicon ion complex thus obtained was transferred to a 250ml beaker and then adjusted in pH to a lower value (≤ 1.5) with 37% hydrochloric acid to determine its stability. In about 20 days of observation, the organized silicon ion complex was maintained in a stable state. This testing was to determine whether the organized silicon ion complex was able to exist as ions in the environment similar to the human gastric acid.
(6) The pH level of the solution of step (5) was changed back to a value of 13.0 using ammonia. The solution remained stable.

A step of filtration is necessary in the process of preparing a silicon ion complex organized with a carboxylic acid. The filtration is to filter out the impurities produced by the pH shock possibly occurring during the dissolution and those remaining in the substances. The filtration as used herein may include precipitate filtration using the supernatant after precipitation, or filter filtration.

In addition, sterilization may be necessary in the preparation process. The sterilization as use herein may include at least one sterilization method selected from UV sterilization, high-temperature sterilization, chlorination, gaseous dioxide sterilization, and ozone sterilization.

In order to adjust the pH of the organized silicon ion complex composed of citric acid to weak acid or weak alkali, carboxylic acids, sodium carbonate, sodium hydrogen carbonate, sodium hydroxide, or amino acids was used, and the adjusted pH value ranged from 1 to 13.

This process made the organized silicon ion complex available for use in water, beverages, or the like. But, the organized silicon ion complex was less stable than that using tartaric acid.

### [Example 2] Experiment for Preparation of Organized Metasilicate Ion Complex Using Malic Acid

An organic ionization testing was performed using metasilicate nonahydrate (Na₂SiO₃·9H₂O).

The procedures were performed as follows.
(1) 20 g of malic acid was added into a 500ml beaker.
(2) Sodium carbonate was used to adjust the pH to a value of 8.0 (alkaline); this pH adjustment was to prevent insoluble impurities from produced by a pH shock, due to the metasilicate dissolving easily under neutral-to-alkali conditions without pH shock.
(3) 20 g of silicate nonahydrate (Na₂SiO₃·9H₂O) was slowly dissolved under mechanical agitation; water absorption and endothermic reaction occurred during the dissolution process. The dissolution rate of the silicate increases with an increase in the dissolution temperature. It was considered more desirable to slowly dissolve the silicate at a low temperature in order to increase the binding force to the organic acid.
(4) The organized silicon ion complex thus obtained was stabilized in the form of a clear solution. 20 g of the complex was sufficiently dissolved in 500 ml of the solution and maintained in an ionic state; when reduced to the amount of pure silicon ions, the silicon ion content amounted to about 3.954 g/L.
(5) 100 ml of the organized silicon ion complex thus obtained was transferred to a 250ml beaker and then adjusted in pH to a lower value (≤ 1.5) with 37% hydrochloric acid to determine its stability. In about 20 days of observation, the organized silicon ion complex was maintained in a stable state.
(6) The pH level of the solution of step (5) was changed back to pH 13.0 using ammonia. The solution was maintained stable.

The organized silicon ion complex of Example 2 was also less stable than that using tartaric acid.

### [Example 3] Experiment 1 for Preparation of Organized Metasilicate Ion Complex Using Tartaric Acid

An organic ionization testing was performed using metasilicate nonahydrate (Na₂SiO₃·9H₂O).

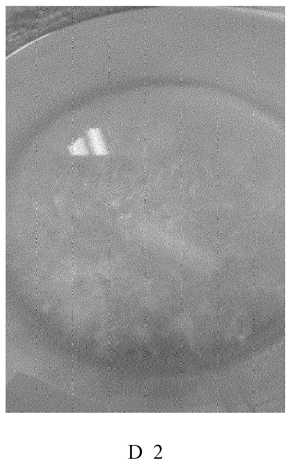

The procedures were performed as follows.
(1) 40 g of tartaric acid was added into a 500ml beaker.
(2) The procedure of pH adjustment was omitted.
(3) 40 g of silicate nonahydrate (Na₂SiO₃·9H₂O) was slowly dissolved under mechanical agitation.
(4) The organized silicon ion complex thus obtained was stabilized in the form of a clear solution. 40 g of the complex was sufficiently dissolved in 500 ml of the solution and maintained in the ionic state; when reduced to the amount of pure silicon ions, the silicon ion content amounted to about 7.908 g/L. Further research is needed as to why such a large amount of silicate was able to dissolve when using tartaric acid.
(5) 100 ml of the organized silicon ion complex thus obtained was transferred to a 250ml beaker and then adjusted in pH to a lower value (≤ 1.5) with 37% hydrochloric acid to determine its stability. In about 20 days of observation, the organized silicon ion complex was maintained in a stable state.
(6) The pH level of the solution of step (5) was changed back to pH 13.0 using ammonia. The solution was maintained stable.
(7) The silicon ion complex solution prepared using tartaric acid was made into a saturated solution at room temperature and observed to determine whether it was subjected to gelation. The solution did not form a silica gel, but produced a precipitate having a new needle-shaped structure (Refer to FIG. 2).

### [Example 4] Experiment 2 for Preparation of Organized Metasilicate Ion Complex Using Tartaric Acid

An organic ionization testing was performed using metasilicate nonahydrate (Na₂SiO₃·9H₂O).

This experiment has an object to determine whether gelation occurs in a saturated solution of the organized metasilicate ion complex and to identify the crystal structure of the complex in the saturated solution.

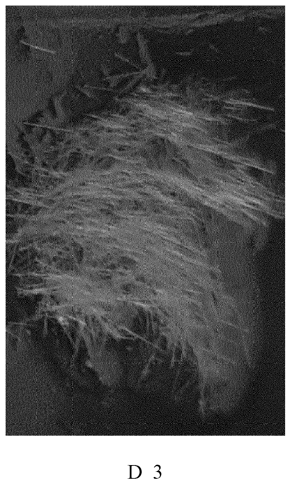

The procedures were performed as follows.
(1) 60 g of tartaric acid was added into a 500ml beaker; its complete dissolution was confirmed by observation with naked eye.
(2) The procedure of pH adjustment was omitted.
(3) 60 g of silicate nonahydrate (Na₂SiO₃·9H₂O) was slowly dissolved under mechanical agitation; water absorption and endothermic reaction occurred during the dissolution process.
(4) The silicate was completely dissolved and partly precipitated in the saturated solution. The precipitate thus obtained had a needle-shaped structure rather than the form of not-dissolved silicate. It was a structure that looked like a fibrous substance possibly found in vegetable fibers. This precipitate was able to re-dissolve (Refer to FIG. 3).

### [Example 5] Experiment for Preparation of Organized Metasilicate Ion Complex Using Glycine

An organic ionization testing was performed using metasilicate nonahydrate (Na₂SiO₃·9H₂O) as follows.
(1) 20 g of glycine was added into a 500ml beaker; its complete dissolution was confirmed by observation with naked eye.
(2) Sodium carbonate was used for adjusting the pH to a value of 8.0. Glycine, which is neutral, can be easily changed into alkali by adding a small amount of sodium carbonate.
(3) 20 g of silicate nonahydrate (Na₂SiO₃·9H₂O) was slowly dissolved under mechanical agitation; water absorption and endothermic reaction occurred during the dissolution process.
(4) The silicate was completely dissolved to have a pH value of 12.25. This pH level can be lowered with citric acid, malic acid, or tartaric acid to make the solution into a slightly alkaline drinking water.

In the preparation of the organized silicon ion complex using a carboxylic acid, it is possible to add minerals such as cations of magnesium, potassium, iron, manganese, sodium, zinc, sulfur, calcium, phosphorus, or titanium or zirconium. In this regard, titanium and zirconium are preferably added in the form of organized ions that are stable against pH changes in an aqueous solution.

Other mineral components as used herein are substances susceptible to ionization in an aqueous solution; cations obtained by dissolution of substances, such as calcium oxide (CaO), calcium hydroxide (Ca(OH)₂), calcium ascorbate (Ca(C₆H₇O₆)₂), calcium carbonate (CaCO₃), calcium chloride (CaCl₂), calcium magnesium acetate, calcium acetate (C₄H₆CaO₄), magnesium acetate (C₄H₆MgO₄), magnesium carbonate-magnesium hydroxide-5 hydrate ((MgCO₃)₄·Mg(OH)₂·5H₂O), magnesium chloride (MgCl₂), magnesium citrate, magnesium hydroxide (Mg(OH)₂), magnesium oxide (MgO), potassium acetate (CH₃COOK), potassium chloride (KCl), potassium citrate, potassium hydroxide (KOH), potassium carbonate (K₂CO₃), potassium hydrogen carbonate (KHCO₃), sodium hydroxide (NaOH), sodium carbonate (Na₂CO₃), sodium hydrogen carbonate (NaHCO₃), sodium acetate (CH₃COONa), sodium citrate, iron oxide, iron chloride, zinc chloride, manganese oxide, sodium phosphate, and trisodium phosphate. A variety of other water-soluble substances can also be used to obtain the cations.

Before addition, these cations are preferably dissolved in an aqueous solution of a dicarboxylic acid or a tricarboxylic acid, more preferably in an aqueous solution of a tricarboxylic acid.

The silicon ion complex organized with a carboxylic acid thus obtained is available for various use purposes. For example, it can be used for various applications, including drinking water suitable for drinking water quality standards, amino acid beverages, mineral beverages, amino acid-mineral composite beverages, pharmaceutical compositions for oral administration or injection to supply silicon minerals to animal and human bodies, breads, confections, fertilizers for plant growth, cleaning agents, cosmetics, and medical ointments.

The cleaning agents can be prepared by adding a certain portion of the silicon ion complex organized with a carboxylic acid according to the present invention to shampoos, conditioners, face cleansing solutions, soaps, dish detergents, laundry detergents, toothpaste, etc. and used as cleansing agents for humans and other animals.

### [Example 6] Plating Experiment

The silicon ion complex using a tartaric acid as prepared in Example 3 was diluted with 1 L of water to lower the sodium silicate concentration per liter by 50%, and then analyzed in regards to the electrochemical ion behavior.

The conventional titanium or zirconium, even though organized (chelated) and made into ions, was available for electrochemical use.

1 L of water was added to the solution of Example 3 (ingredient 1) to make 2 L of a diluted solution. 0.1 g of potassium gold cyanide (ingredient 2) and a trace amount (0.01 g or less) of sodium lauryl sulfate (ingredient 3) were added to 250 ml of the diluted solution to prepare 1 L of a gold plating solution.

With an iridium-coated titanium electrode used as an anode, 1.5V DC current was applied to perform a gold plating on a metallic accessory part, of which the surface was inspected. If insufficient, gold was supplemented in small portions, and the changes in color and texture were measured.

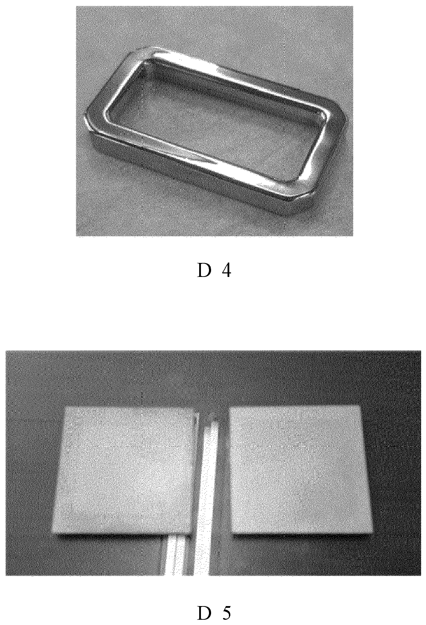

This experiment was to determine from the texture and color of the surface whether silicon had an ion behavior, although the thickness of silicon was not measurable with a plating thickness measuring device. The measurement results are presented in FIG. 4.

The gold-plated surface had a slight blue sensation, expressing the texture as if it was coated with a glass film, and the yellow color of gold was slightly darker.

This result confirmed that the silicon ion complex could be used for a plating solution, such as a gold plating solution. This phenomenon appeared similarly in most of electroplating solutions. Therefore, the silicon ion complex of the present invention can be used in the process of metal reduction and deposition in the electrochemical field, plating, to add the texture of glass, so it is expected to be used for various applications.

### [Example 7] Substitution Experiment

In addition, a substitution experiment was performed to determine whether the silicon ion complex prepared according to the present invention and present as an ion had a substitution ability.

The reason of this experiment was that it was needed to confirm whether silicon as a metalloid caused a substitution reaction like other metals while in an ionic state.

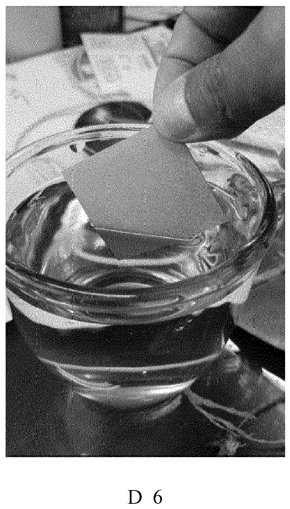

In the substitution experiment, 1 L of water was added to the solution prepared in Example 3 to make 2 L of a diluted solution, and various metals were immerged in 250 ml of the diluted solution. As a result, the stainless steel surface exhibited surface purification ability and texture like a glass film coating (Refer to FIG. 5). When the surface was viewed from various angles in the sunlight, it had a light scattering like the pieces of broken glass and a little texture of glass. In addition, the surface was hydrophobic and impervious to water (Refer to FIG. 6).

It was therefore confirmed that the present invention is not only performing the function of a mineral, but also available as metalloid ions in various applications including electrochemistry (plating), as the metals of titanium and zirconium organized into ions were electrochemically available.

The terminology used herein is for the purpose of describing an embodiment only and is not intended to be limiting of an exemplary embodiment. As used herein, the singular forms are intended to include the plural forms as well, unless the context clearly indicates otherwise. It will be further understood that the terms "comprises" and/or "comprising" when used in this specification, specify the presence of stated form, numeral, step, operation, member, element, and/or a combination thereof but do not preclude the presence or addition of one or more other forms, numerals, steps, operations, members, elements, and/ or combinations thereof.

For example, the organic acid, amino acid, or carboxylic acid used in the present invention is not necessarily limited to only the organic acid, amino acid, or carboxylic acid, but may include sodium compounds, potassium compounds, or ammonium compounds containing such components.

Although the exemplary embodiments of the present invention have been described, it is understood that the present invention should not be limited to these exemplary embodiments but various modifications can be made within the scope of the present invention as hereinafter claimed.

## Claims

1. A method for preparing a silicon ion complex organized with a carboxylic acid, comprising:
(1) dissolving at least one carboxylic acid selected from a dicarboxylic acid and a tricarboxylic acid to prepare an aqueous solution of carboxylic acid;
(2) adjusting the pH of the aqueous solution of carboxylic acid to a value ranging from 1.0 to 13.0 to make the aqueous solution acidic or basic;
(3) dissolving a water-soluble silicate compound in the pH-adjusted aqueous solution of carboxylic acid; and
(3) selectively adding a cation.

2. The method according to claim 1, wherein the dicarboxylic acid in step (1) is an amino acid.

3. The method according to claim 1, wherein adjusting the pH of the aqueous solution to a value ranging from 1.0 to 13.0 to make the solution acidic or basic in step (2) is adding any one of carboxylic acid, sodium carbonate (Na₂CO₃), sodium hydrogen carbonate (NaHCO₃), sodium hydroxide (NaOH), and amino acid.

4. The method according to claim 1, wherein the water-soluble silicate compound dissolved in step (3) is at least one selected from a water-soluble silicate containing sodium, potassium, or calcium in addition to silicon and a water-soluble silicate complex prepared by selectively adding a mineral in the preparation of the water-soluble silicate, the water-soluble silicate including sodium silicate, potassium silicate, or calcium silicate.

5. The method according to claim 4, wherein the water-soluble silicate complex prepared by selectively adding a mineral in the preparation of the water-soluble silicate is a water-soluble silicate complex prepared by adding at least one selected from sodium carbonate, potassium carbonate, sodium triphosphate, sodium pyrophosphate, sea salt, magnesium carbonate, calcium carbonate, magnesium oxide, calcium oxide, iron oxide, or manganese oxide.

6. The method according to claim 1, wherein the cation selectively added in step (4) is selected from the cations of magnesium, potassium, iron, manganese, sodium, zinc, sulfur, calcium, phosphorus, titanium, or zirconium.

7. The method according to claim 6, wherein selectively adding a cation is dissolving a substance containing the cation in an aqueous solution of a dicarboxylic acid or a tricarboxylic acid.

8. The method according to claim 1, further comprising:
performing sterilization after step (4) by using at least one method selected from UV sterilization, high-temperature sterilization, chlorination, gaseous dioxide sterilization, or ozone sterilization.

9. A silicon ion complex organized with a carboxylic acid prepared by the method according to claim 1.
